**Europäisches Patentamt**

(19) **European Patent Office**

**Office européen des brevets**

(11) Numéro de publication: **0 098 224**
**B1**

(12) **FASCICULE DE BREVET EUROPEEN**

(45) Date de publication du fascicule du brevet:
**07.01.87**

(21) Numéro de dépôt: **83401336.9**

(22) Date de dépôt: **28.06.83**

�[51] Int. Cl.⁴: **A 61 F 2/30**

(54) **Nouvelle prothèse osseuse et son obtention.**

(30) Priorité: **29.06.82 FR 8211412**

(43) Date de publication de la demande:
**11.01.84 Bulletin 84/2**

(45) Mention de la délivrance du brevet:
**07.01.87 Bulletin 87/2**

(84) Etats contractants désignés:
**AT BE CH DE FR GB IT LI LU NL SE**

(56) Documents cités:
**FR - A - 2 290 882**
**GB - A - 2 069 340**
**US - A - 2 719 522**
**US - A - 3 740 769**

(73) Titulaire: **Lord, Gérald, 4, rue Thiers, F-75116 Paris (FR)**

(72) Inventeur: **Lord, Gérald, 4, rue Thiers, F-75116 Paris (FR)**

(74) Mandataire: **Phélip, Bruno et al, c/o Cabinet Harlé &
Phélip 21, rue de la Rochefoucauld, F-75009 Paris (FR)**

## Description

La présente invention concerne le domaine des prothèses osseuses. Elle a pour objet une nouvelle prothèse et son obtention.

On connaît déjà la technique générale d'implantation de pièces métalliques destinées à remplacer ou à consolider des os ou des articulations osseuses. Bien entendu, la forme de l'implant doit être adaptée à la partie osseuse concernée. C'est le cas, par exemple, des prothèses destinées aux articulations de la hanche ou du genou.

On a d'abord utilisé des implants métalliques en les fixant dans l'os vivant par un ciment, notamment par un ciment à base de résine acrylique. Cette technique de fixation avec un ciment entraîne de nombreux inconvénients, parmi lesquels on peut signaler un défaut de tenue de la prothèse en raison des risques de fractionnement du ciment, ainsi qu'une intolérance par l'organisme et le tissu osseux du corps étranger que constitue le ciment.

C'est pourquoi on a proposé des prothèses dont la mise en place ne nécessitait pas l'utilisation de ciment. A cet effet, on a rendu la surface de l'implant rugueuse ou poreuse, de manière à permettre le développement du tissu osseux à la surface de la prothèse elle-même, et d'en assurer ainsi l'ancrage dans l'os. Les inconvénients de la technique de fixation au ciment sont pratiquement éliminés en totalité par cette technique, mais celle-ci peut encore être perfectionnée. A titre illustratif, on peut estimer que l'utilisation de ciment fournit, dans le cas des prothèses de la hanche, plus de 25% d'échecs, en raison du descellement de l'implant dans l'os. Ce pourcentage d'échecs est sensiblement réduit lorsqu'on donne à l'implant une surface poreuse ou rugueuse, mais dans au moins 10% des cas, pour des raisons très variées, il est nécessaire d'intervenir à nouveau afin de remplacer la prothèse. Mais, dans ce cas, la prothèse ne peut pas simplement être extraite car, en raison de l'effet d'ancrage extrêmt résistant, procuré par le développement du tissu osseux, il faut, pour remplacer la prothèse, effectuer une intervention délicate avec découpe de l'os pour sortir la prothèse.

A titre de documents antérieurs illustrant l'état de la technique, on peut citer le DE-C 837 294, le FR-A 2 194 123, le US-A 3 905 047 et le FR-A 2 356 465. L'enseignement de ces divers brevets peut être brièvement résumé comme suit.

Le DE-C 837 294 a pour objet une prothèse dont l'axe (ou queue) présente une surface extérieure cannelée dans un sens général parallèle à l'axe moyen. Aucune information précise n'est donnée sur les cannelures dont la forme, le nombre et l'importance peuvent être quelconques. De même, leur profil peut être à angle aigu ou arrondi. En fait, ces cannelures ne peuvent pas assurer un effet de rétention suffisant du tissu osseux et, de plus, le profil longitudinal rectiligne de ces cannelures ne permet pas une extraction de la queue de prothèse dans des conditions satisfaisantes.

Le FR-A 2 194 123 propose une prothèse destinée à être fixée sans ciment, et comportant une surface extérieure poreuse. Une fois mise en place, une telle prothèse ne peut pas être extraite. En outre, les conditions d'obtention d'une telle prothèse conduisent à un risque de corrosion de la surface. Celle-ci est également rendue plus fragile ce qui, sous l'effet des contraintes élevées, peut provoquer des zones de faiblesse et aboutir finalement à la fracture de la prothèse.

Le brevet US-A 3 905 047 concerne une prothèse comportant des irrégularités de surface. Une fois mise en place, une telle prothèse ne peut pas être extraite par un simple effort de traction, en raison du développement du tissu osseux, qui constitue l'une des caractéristiques revendiquées par le brevet.

Le FR-A 2 356 465 concerne notamment une prothèse comportant à sa surface extérieure un revêtement constitué de billes ou sphérules. Celles-ci sont réparties au hasard, afin d'assurer un solide effet d'ancrage. Une telle prothèse ne peut, sans intervention particulière, être extraite en cas d'accident.

On connait également de nombreuses prothèses dont la mise en place nécessite l'utilisation d'un ciment. Les queues de certaines de ces prothèses sont conformées en présentant des gorges ou des parties longitudinales en creux, essentiellement afin d'alléger la prothèse et d'assurer une bonne insertion dans le ciment. C'est le cas, notamment, de la prothèse décrite dans le document GB-A 2 069 340, qui décrit une prothèse dans laquelle la queue de la prothèse est fixée dans l'os, par l'intermédiaire du ciment, de manière à ne pas pouvoir se déplacer longitudinalement ou en rotation, par rapport à l'os dans lequel la prothèse est insérée.

On constate donc, qu'à l'heure actuelle, il n'existe pas, à la connaissance du demandeur, des prothèses osseuses pouvant être mises en place sans ciment, capables d'être retenues efficacement dans le tissu osseux, après développement de celui-ci, et en même temps d'être extraites, en cas de nécessité, sans être obligé de pratiquer une intervention délicate. L'invention a pour objet une nouvelle prothèse qui remplit simultanément toutes ces exigences.

L'invention a également pour objet l'obtention d'une telle prothèse, dans des conditions industrielles permettant d'aboutir à un produit possédant une résistance mécanique très supérieure à celle des prothèses actuellement connue. En particulier, le procédé d'obtention ne provoque aucun affaiblissement de la surface de la prothèse.

L'invention a pour objet une prothèse osseuse destinée à être fixée sans ciment, et présentant sur sa partie déstinée à être enfoncée l'os une surface extérieure cannelée dans un sens généralement parallèle à son axe moyen, ladite prothèse étant caractérisée en ce que:

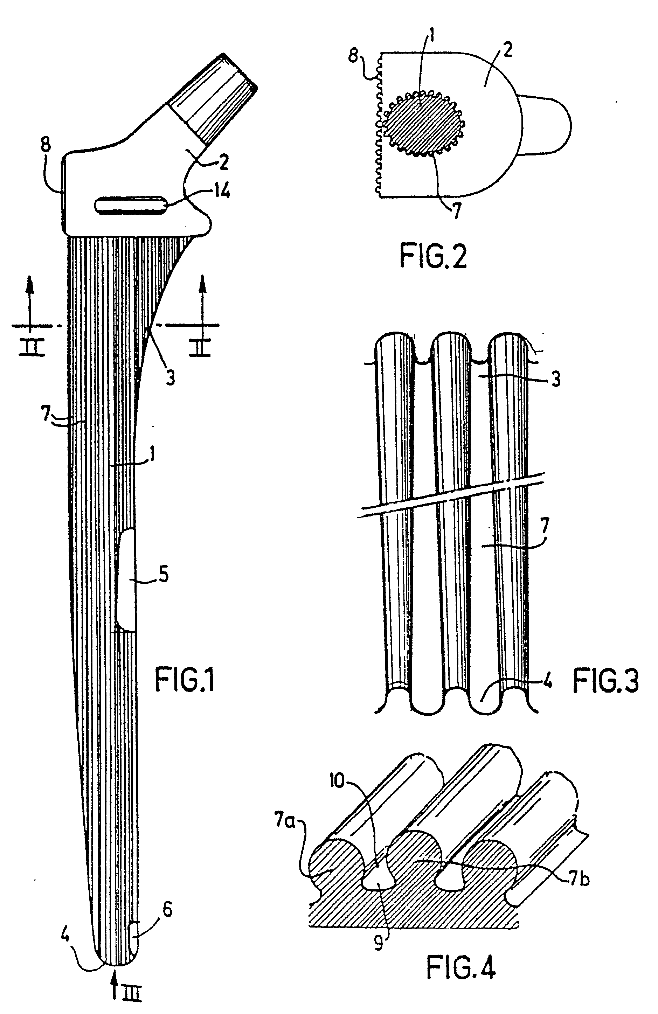

– la surface intérieure des cannelures présente un profil en section transversale comportant une partie rétrécie (10 fig. 4) qui s'épanouit vers le fond (9) de la cannelure, ce profil assurant la rétention du tissu osseux développé à l'intérieur de la cannelure;

– la surface intérieure longitudinale des cannelures, non cylindrique, présente une forme allant en s'amincissant depuis l'extrémité dite basse (4) de la prothèse, c'est à dire celle qui s'enfonce le plus profondément dans l'os jusqu'à l'autre extrémité (3) dite haute, la prothèse pouvant ainsi être extraite du tissu osseux, par un mouvement de traction.

La prothèse selon l'invention constitue un perfectionnement à l'objet du DE-C 837 294, en procurant, en combinaison, l'effet d'ancrage recherché pour le tissu osseux et la faculté d'extraction. Le profil transversal des cannelures doit procurer un pouvoir élevé de rétention du tissu osseux. Contrairement à l'enseignement du DE-C 837 294, ce profil ne doit donc pas être ouvert, mais comporter une partie rétrécie, de manière que le tissu osseux se développant puisse être emprisonné au fond de la cannelure. A cet effet, le profil transversal peut être curviligne ou circulaire, ou une association de ces deux types de profils, les arcs pouvant être raccordés par des parties rectilignes. En combination avec ce profil transversal, les cannelures pratiquées à la surface externe de la prothèse selon l'invention, comportent dans le sens longitudinal une légère conicité entre l'extrémité enfoncée dans l'os et celle qui, le cas échéant, doit être saisie pour l'extraction. Les résultats avantageux de l'invention sont obtenus avec des conicités dont la valeur peut être relativement faible. Une pente de 1% à 1‰ s'est révélée convenable.

L'invention est applicable à toutes sortes d'implants métalliques pouvant servir de prothèses, ou utilisés pour le traitement des os. On obtient des résultats particulièrement intéressants dans le cas des prothèses de la hanche ou du genou, et d'une façon générale avec toutes les prothèses comportant une pièce en forme de tige. Une telle tige peut être rectiligne ou présenter une certaine courbure, qui lui permet de mieux épouser la forme de l'os dans lequel elle est destinée à être ancrés. De même, la surface extérieure de la tige ne se présente pas nécessairement sous forme d'un cylindre régulier. Il est même de nombreux cas avantageux, notamment pour satisfaire aux exigences de la résistance des matériaux, de conformer la tige en lui donnant une épaisseur progressivement augmentée, au moins dans sa partie haute. Certaines des cannelures s'étendent sur toute la longueur de la tige, et certaines autres peuvent, si elles partent de la partie haute, aboutir à un bord de la tige sans aller jusqu'à son extrémité.

Sous un autre aspect, l'invention a pour objet un procédé pour l'obtention d'une pièce de prothèse cannelée par la technique générale de fonderie en cire perdue, dans laquelle on réalise un modèle en cire correspondant à la pièce à fabriquer, on effectue un moulage réfractaire sur ledit modèle pour constituer un moule, et on coule du métal liquide dans ce moule pour obtenir la pièce désirée, ledit procédé étant caractérisé en ce que, avant le moulage réfractaire, on met en place dans les cannelures du modèle en cire, des baguettes dont le profil transversal correspond à celui des cannelures de la pièce finale et qui présentent une légère conicité d'une extrémité à l'autre, de manière à obtenir, après coulage du métal dans le moule réfractaire, une pièce avec des cannelures dont la surface intérieure longitudinale non cylindrique, présente une forme allant en s'amincissant depuis l'extrémité basse jusqu'à l'extrémité haute.

Dans le cas, par exemple, d'une pièce de prothèse comportant une queue, on réalise tout d'abord la queue en cire aux dimensions de la pièce à reproduire, en prévoyant à la périphérie de la queue, des rainures concaves parallèles à l'axe. On fabrique par ailleurs, des baguettes, par exemple en polystyrène ou en cire, ayant la longueur des cannelures à réaliser, et présentant une légère conicité d'une extrémité à l'autre. Le profil des baguettes, en section transversale, correspond à celui des cannelures de la pièce finale. Ces baguettes sont assemblées, en particulier par collage, dans les rainures concaves de la queue. On a ainsi réalisé un modèle en cire de la pièce à fabriquer. On procède ensuite de manière connue, à l'enrobage réfractaire de ce modèle en cire, pour réaliser un moule réfractaire et la pièce finale est obtenue par coulage du métal liquide dans un tel moule, duquel toute cire a été préalablement enlevée.

Sous un autre aspect, qui correspond à un mode de réalisation préféré, l'invention a pour objet un procédé pour l'obtention d'une pièce de prothèse cannelée, caractérisé en ce qu'on réalise par la technique générale connue de fonderie en cire perdue, une ébauche métallique lisse présentant une légère surépaisseur par rapport aux dimensions transversales de la pièce à fabriquer, en ce qu'on traite ladite ébauche successivement par forgeage à chaud puis par forgeage à froid, jusqu'à obtention des dimensions définitives de la pièce désirée, et en ce qu'on réalise sur celle-ci, par électro-usinage, des cannelures longitudinales ayant le profil transversal desiré et dont la surface interieure longitudinale, non cylindrique, présente une forme allant en s'amincissant depuis l'extrémité basse jusqu'à l'extrémité haute.

Dans un tel procédé, on produit tout d'abord par la technique de fonderie en cire perdue, un modèle de pièce, par exemple de queue, dont la surface extérieure est lisse et qui présente une légère surépaisseur par rapport aux dimensions transversales de la pièce à obtenir. A titre d'exemple concret, pour une queue d'environ 15 mm de diamètre, une surépaisseur de 1 mm par face est convenable. Les ébauches brutes de fonderie sont ensuite reprises par forgeage à chaud pour obtenir une pièce intermédiaire, encore en légère surépaisseur (dans l'exemple chiffré ci-dessus celle-ci peut être d'environ 0,50 mm). Le calibrage final donnant les dimensions définitives est effectué par forgeage à froid. A partir de cette queue forgée, à surface extérieure lisse, on réalise les cannelures par enlèvement de métal, en utilisant la technique de l'électro-usinage. A cet effet, on utilise un outil qui est déplacé dans le sens longitudinal de la queue, pour y creuser les cannelures. On peut opérer par passes successives, à partir d'outils ayant des profils différents. Dans une variante, qui est préférée du point de vue économique, on utilise un seul outil en une passe, pour parvenir au profil transversal de cannelures désiré, à savoir permettant un effet de rétention optimale du tissu osseux lors du développement de celui-ci.

Le procédé qui vient d'être décrit est le plus intéressant dans la pratique, En effet, à partir de l'ébauche venue de fonderie, les étapes de forgeage à chaud et à froid améliorent très sensiblement les caractéristiques mécaniques de la pièce. La technique d'électro-usinage respecte intégralement les propriétés mécaniques, et peut être mise en œuvre sur des équipements usuels, qui n'ont besoin que de comporter un outil répondant aux nécessités de l'invention. L'invention sera maintenant illustrée sans être aucunement limitée, à l'aide de la description ci-après, qui concerne une prothèse destinée à être insérée dans le fémur (prothèse de hanche), en référence aux dessins annexés sur lesquels:

Fig. 1 est une vue de face de la prothèse;
Fig. 2 est une coupe faite selon la ligne II-II de la figure 1;
Fig. 3 est une vue de l'intérieur d'une cannelure faite suivant la flèche III de la figure 1;
Fig. 4 montre à une échelle agrandie, un profil de cannelure;
Fig. 5 est une figure analogue à celle de la figure 4, montrant une variante de profil;
Fig. 6 est un schéma illustrant un procédé de fabrication de la prothèse selon l'invention;
Figs. 7 et 8 sont des schémas illustrant un autre procédé préféré de fabrication de la prothèse selon l'invention.

Les figures 1 et 2 représentent, d'une manière schématique, une pièce de prothèse destinée à être insérée dans le fémur. D'une manière générale, elle se compose de deux parties, une queue (1) et une partie (2) que, par commodité, on appelera «col». Dans l'exemple choisi, la queue (1) mesure environ 200 mm de long, et sa dimension transversale, approximativement, est de 15 mm. La figure 2 montre d'une manière plus claire, en coupe, la forme légèrement elliptique de la queue (1). L'extrémité (4) s'enfonce le plus profondément dans l'os du fémur. L'extrémité (3) est conformée comme le représente la figure 1, de manière à offrir une résistance mécanique maximale. La queue (1) comporte par ailleurs des évidements dont deux ont été représentés (5,6).

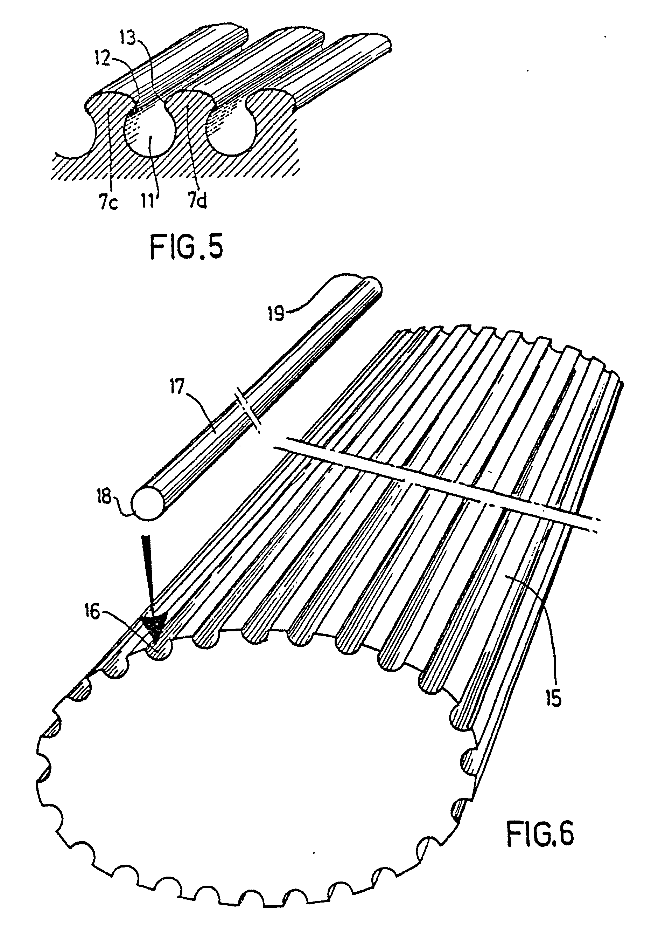

Conformément à l'invention, la queue (1) comporte des cannelures (7). Certaines des cannelures (7), en particulier la partie médiane, s'étendent sur toute la longueur de la queue (1). D'autres cannelures, en particulier au voisinage de la partie supérieure (3) ou des évidements (5,6) ne se prolongent pas jusqu'à l'extrémité (4), mais s'arrêtent au bord externe correspondant de la queue. On voit également aux figures 1 et 2, qu'une partie (8) du col, se trouvant en prolongement de la queue (1) est également cannelée. Cette partie (8) se trouve limitée à une face du col (2). La particularité essentielle de l'invention est que les cannelures (7,8) ont un profil transversal capable d'assurer une rétention efficace du tissu osseux lorsqu'il se développe après enfoncement de la prothèse. On a représenté aux figures 4 et 5, des exemples de profils convenables. A la fig. 4, on voit deux cannelures adjacentes (7a, 7b), délimitant entre elles un fond (9) ou surface intérieure de cannelure. On voit que, à la figure 4, la surface intérieure (9) est délimitée par une courbe qui se rétrécit comme indiqué en (10), avant de s'épanouir pour former la cannelure proprement dite. C'est la conformation de la surface intérieure qui assure l'effet d'ancrage et le pouvoir de rétention du tissu osseux. Il va sans dire que le dessin de la figure 4 est fortement agrandi (grossissement environ 10 fois).

La fig. 5 représente une variante de réalisation avec deux cannelures adjacentes (7c, 7d). Le fond (11) de cannelure présente deux bossages (12, 13) se rapprochant à la partie supérieure. Dans ce cas également, l'effet de rétention recherché est assuré.

Selon une autre caractéristique fondamentale de l'invention, qui se combine à celle concernant le profil transversal de la cannelure, le profil longitudinal de celle-ci n'est pas cylindrique, mais forme au contraire une dépouille. Comme l'illustre la figure 3, la surface intérieure d'une cannelure (7) va en s'amincissant depuis l'extrémité (4) enfoncée la plus profondément dans l'os, jusqu'à l'extrémité (3) voisine du col (2). Il est clair que la représentation de la figure 3 est schématique. Dans la pratique, avec les dimensions de queue précédentes (à savoir: longueur 200 mm, diamètre 15 mm) la dimension transversale d'une cannelure au voisinage de l'extrémité (4) est par exemple de 1,5 mm, et elle est de 1,3 mm au voisinage de l'extrémité (3). Ce profil évasé de haut en bas, permet une extraction de la prothèse en cas de nécessité.

En vue de l'extraction, on peut prévoir sur le col (2), une paire d'encoches (14) (voir fig. 1) permettant au chirurgien d'insérer un outil en vue de l'extraction de la prothèse.

On a illustré schématiquement à la fig. 6, un procédé d'obtention d'une queue de prothèse, du genre représenté aux figures 1 à 5. Ce procédé fait appel à la technique générale de fonderie en cire perdue. La figure 6 représente la queue (15) en cire qui est réalisée selon les dimensions de la pièce à produire. Elle présente sur toute sa périphérie des rainures concaves (16) parallèles à l'axe général de la queue (15). On réalise par ailleurs des baguettes (17) en polystyrène ou en cire, présentant un profil transversal analogue aux cannalures des figures 4 et 5, et un profil longitudinal en dépouille. Ainsi, leur diamètre à l'extrémité (18) est supérieur à celui de l'extrémité (19). Ces baguettes sont individuellement mises en place par collage dans les rainures concaves (16) de la queue en cire. On procède ensuite de manière connue, en enrobant de matériau réfractaire le modèle en cire obtenu conformément à la représentation de la figure 6. A partir du moule réfractaire, on peut couler du métal liquide pour réaliser la pièce de prothèse finale.

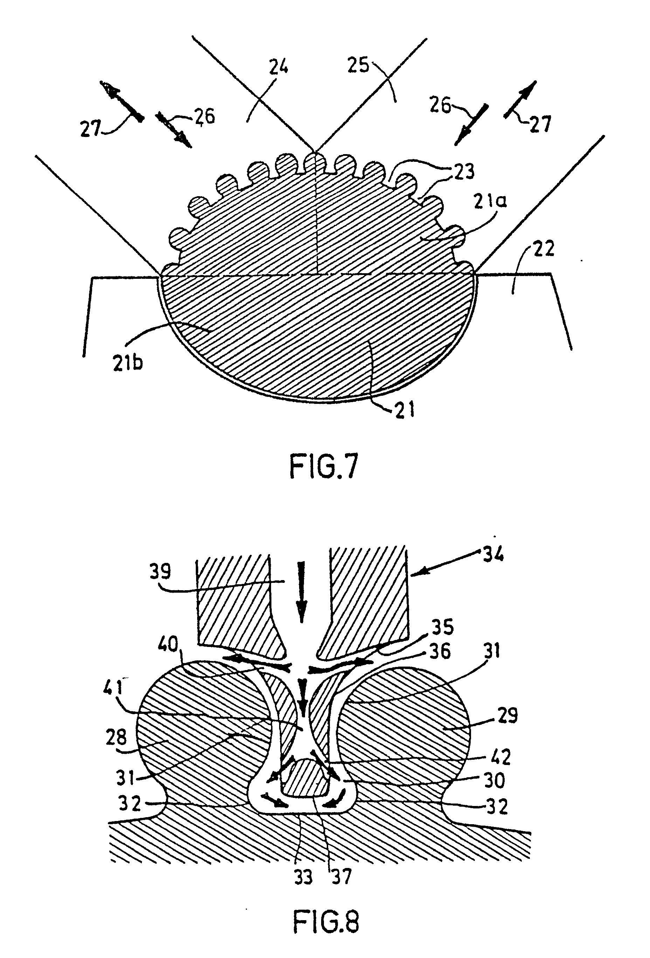

On préfère fabriquer la prothèse selon l'invention par un procédé dans lequel on réalise tout d'abord, par la technique de fonderie en cire perdue, un modèle de queue dont la surface extérieure est lisse, et dont les dimensions transversales sont légèrement agrandies par rapport à celles de la pièce finale à obtenir. Avec les chiffres indiqués précédemment (longueur de la queue de 200 mm et diamètre de 15 mm), la surépaisseur du modèle de cire peut être d'environ 1 mm sur chaque face, soit 2 mm en tout. On réalise ensuite une ébauche de queue métallique présentant une telle surépaisseur. L'ébauche est alors reprise par forgeage à chaud pour obtenir une pièce intermédiaire ayant, par rapport à la pièce finale, une surépaisseur moindre, par exemple d'environ 0,50 mm par face. Le calibrage final est obtenu par forgeage à froid, ce qui améliore considérablement les caractéristiques mécaniques de la prothèse. La forme définitive cannelée de la prothèse selon l'invention est obtenue par enlèvement de métal par une technique d'électro usinage. Celle-ci a été illustrée schématiquement, aux figures 7 et 8. La figure 7 montre en coupe la queue 21 de la prothèse à fabriquer. Celle-ci est maintenue horizontalement dans un outillage 22 de fixation qui ne laisse apparaître que la moitié supérieure 21a de la queue 21. Les rainures ou cannelures longitudinales 23 sont obtenues grâce à la mise en action d'une paire d'électrodes 24, 25. L'extrémité active de chaque électrode couvre la moitié de la partie supérieure 21a de la queue, de manière que cette partie supérieure 21a soit couverte en totalité par les électrodes, comme représenté à la figure 7. Pour l'électroérosion ou électro-usinage, chaque électrode 24, 25 est approchée de la partie 21a (flèches 26) dans un mouvement convergent, après quoi les deux électrodes se retirent (flèches 27). La pièce (queue 21) est ensuite retournée et la même opération s'effectue sur l'autre moitié 21b.

La fig. 8 montre d'une manière agrandie la constitution d'un élément d'électrode 24, 25. On voit au dessin deux cannelures adjacentes 28, 29 séparées par une gorge 30. Dans l'exemple choisi, la gorge 30 présente un profil résultant de la combinaison d'arcs de cercle 31 et 32, de courbure opposée. Le fond de la gorge est constitué par un méplat 33.

L'élément 34 de l'électrode, qui est réalisé en cuivre, est conçu pour réaliser l'usinage du profil 31, 32, 33 et, à cet effet, il comporte des parties arquées 35,36 et une partie extrême 37. Des canaux intérieurs sont prévus dans l'élément 34 d'électrode pour permettre le passage d'un électrolyte, comme le représentent les flèches à la figure 8. On voit ainsi que l'élément 34 possède un canal principal 39, deux passages latéraux 40, un canal axial 41, et deux autres passages latéraux 42. Grâce à la conformation de l'élément 34 d'électrode et à la circulation de l'électrolyte (qui peut être de la saumure), on obtient un enlèvement du métal de la queue de prothèse selon le profil désiré pour la gorge 30 entre les cannelures 28, 29 tant en direction transversale qu'en direction longitudinale.

Il va sans dire que les procédés de fabrication décrits ci-dessus ne constituent que de simples exemples. L'homme de lart comprendra que des variantes peuvent être prévues, en particulier pour adapter la technique de fabrication à la nature du matériau de la prothèse.

Ainsi la pièce brute ou ébauche de prothèse peut être également obtenue par forgeage à chaud à partir d'une barre cylindrique de dimensions appropriées. Le forgeage à chaud nécessite un nombre de passes qui dépend de la nature du matériau. A l'issue du forgeage, on obtient une pièce ayant des caractéristiques mécaniques élevées.

Les techniques ci-dessus conviennent bien pour les aciers inoxydables tels que les aciers chrome-cobalt, par exemple celui de nuance HS21. Bien entendu d'autres matériaux peuvent être mis en œuvre, en particulier d'autres alliages métalliques.

## Revendications

1. Prothèse osseuse destinée à être, fixée sans ciment, et présentant sur sa partie déstinée à être enfoncée dans l'os une surface extérieure cannelée dans un sens généralement parallèle à son axe moyen, ladite prothèse étant caractérisée en ce que:
   - la surface intérieure des cannelures présente un profil en section transversale comportant une partie rétrécie (10 figure 4) qui s'épanouit vers le fond (9) de la cannelure, ce profil assurant la rétention du tissu osseux déléloppé à l'intérieur de la cannelure;
   - la surface intérieure longitudinale des cannelures, non cylindrique, présente une forme allant en s'amincissant depuis l'extrémité dite basse (4) de la prothèse, c'est-à-dire celle qui s'enfonce le plus profondément dans l'os, jusqu'à l'autre extremité (3) dite haute, la prothèse pouvant ainsi être extraite du tissu osseux, par un mouvement de traction.

2. Prothèse selon la revendication 1, caractérisée en ce que le profil en section transversale des cannelures est curviligne ou circulaire ou une association de ces deux types de profils, les arcs pouvant être raccordés par des parties rectilignes.

3. Prothèse selon l'une des revendications 1 ou 2, caractérisée en ce que la dimension transversale de l'intérieur d'une cannelure à l'extrémité haute (3) est inférieure à celle de l'extrémité basse (4).

4. Prothèse selon la revendication 3, caractérisée en ce que le rapport entre les dimensions transversales de l'extrémité haute et de l'extrémité basse, est compris entre 0,99 et 0,099.

5. Procédé pour l'obtention d'une pièce de prothèse cannelée, selon l'une quelconque des revendications 1 à 4, par la technique générale de fonderie en cire perdue, dans laquelle on réalise un modèle en cire correspondant à la pièce à fabriquer, on effectue un moulage réfractaire sur ledit modèle pour constituer un moule, et on coule du métal liquide dans ce moule pour obtenir la pièce désirée, ledit procédé étant caractérisé en ce que, avant le moulage réfractaire, on met en place dans les cannelures (16) du modèle en cire, des baguettes (17), par exemple en cire ou en polystyrène, dont le profil transversal correspond à celui des cannelures de la pièce finale et qui présentent une légère conicité d'une extrémité à l'autre, de manière à obtenir, après coulage du métal dans le moule réfractaire, une pièce avec des cannelures, dont la surface intérieure longitudinale, non cylindrique, présente une forme allant en s'amincissant depuis l'extrémité basse jusqu'à l'extrémité haute.

6. Procédé pour l'obtention d'une pièce de prothèse cannelée selon l'une quelconque des revendications 1 à 4, caractérisé en ce qu'on réalise par la technique générale connue de fonderie en cire perdue, une ébauche métallique lisse présentant une légère surépaisseur par rapport aux dimensions transversales de la pièce à fabriquer, en ce qu'on traite ladite ébauche successivement par forgeage à chaud puis par forgeage à froid, jusqu'à obtention des dimensions définitives de la pièce désirée, et en ce qu'on réalise sur celle-ci, par électro-usinage, des cannelures (28, 29) longitudinales, ayant le profil transversal (30) désiré et dont la surface intérieure longitudinale, non cylindrique, présente une forme allant en s'amincissant depuis l'extrémité basse jusqu'à l'extrémité haute.

7. Procédé selon la revendication 6, caractérisé en ce que, lors de l'électro usinage, on réalise au moins une passe à l'aide de deux électrodes (24, 25) travaillant en plongée dans un mouvement (26) convergent et ayant un profil correspondant à celui des cannelures.

8. Procédé selon l'une des revendications 6 ou 7, caractérisé en ce qu'on opère successivement sur les moitiés (21a, 21b) de la queue (21) de prothèse, laquelle est maintenue horizontalement dans un outillage (22).

9. Procédé pour l'obtention d'une prothèse selon l'une quelconque des revendications 1 à 4, par forgeage à chaud à partir d'une barre cylindrique.

**Patentansprüche**

1. Knochenprothese, die zur zementlosen Fixierung bestimmt ist und auf ihrem zum Eintreiben in den Knochen bestimmten Teil eine äussere Oberfläche aufweist, die im allgemeinen parallel zu ihrer Mittelachse kanelliert ist, dadurch gekennzeichnet,
   – dass die innere Oberfläche der Kanellierungen ein Querschnittsprofil aufweist, das einen kleiner werdenden Abschnitt (10 in Figur 4) hat, der sich zum Boden (9) der Kanellierungen hin erweitert, so dass das Querschnittsprofil die Zurückhaltung des im Innern der Kanellierungen gebildeten Knochengewebes sichert,
   – und dass die nichtzylindrische, längliche Innenfläche der Kanellierungen eine Form aufweist, die sich, ausgehend von dem sogenannten unteren Ende (4) der Prothese d.h. dem am tiefsten in den Knochen eingetriebenen Ende bis zu dem anderen, sogenannten oberen Ende (3) der Prothese, verjüngt, so dass die Prothese mittels einer Zugkraft aus dem Knochengewebe herausgezogen werden kann.

2. Prothese nach Anspruch 1, dadurch gekennzeichnet, dass das Querschnittsprofil der Kanellierungen krummlinig oder kreisförmig oder als eine Vereinigung dieser beiden Profiltypen ausgebildet ist, wobei die Bögen durch geradlinige Strecken verbunden sein können.

3. Prothese nach Anspruch 1 oder 2, dadurch gekennzeichnet, dass die innere Querdimension einer Kanellierung am oberen Ende (3) kleiner ist als diejenige am unteren Ende (4).

4. Prothese nach Anspruch 3, dadurch gekennzeichnet, dass das Verhältnis der Querdimension des oberen Endes und des unteren Endes zwischen 0,99 und 0,099 liegt.

5. Verfahren zur Herstellung eines kanellierten Prothesenteiles nach einem der Ansprüche 1 bis 4 durch Anwendung der allgemeinen Giesstechnik mit verlorenem Wachs, wonach man ein dem herzustellenden Werkstück entsprechendes Wachsmodell verwirklicht, von diesem Wachsmodell einen feuerfesten Abguss zur Bildung einer Form herstellt und flüssiges Metall in diese Form giesst, um das gewünschte Werkstück zu erhalten, dadurch gekennzeichnet, dass man vor dem Herstellen des feuerfesten Abgusses in die Kanellierungen (16) des Wachsmodells Stäbe (17) aus z.B. Wachs oder Polystyrol einsetzt, deren Querschnittsprofil demjenigen der Kanellierungen des fertigen Werkstückes entspricht und die eine leichte Konizität von einem Ende zum anderen Ende aufweisen, derart, dass nach dem Giessen des Metalles in die feuerfeste Form ein Werkstück mit den Kanellierungen erhalten wird, deren nichtzylindrische, längliche Innenfläche eine Form aufweist, die sich von dem unteren Ende bis zu dem oberen Ende verjüngt.

6. Verfahren zur Herstellung eines kanellierten Prothesenteiles nach einem der Ansprüche 1 bis 4, dadurch gekennzeichnet, dass man durch allgemein bekannte Giesstechnik mit verlorenem Wachs einen glatten metallischen Rohling verwirklicht, der eine geringfügige Bearbeitungszugabe in Bezug auf die Querabmessungen des herzustellenden Werkstückes aufweist, dass man die Bearbeitungszugabe aufeinanderfolgend durch Warmschmieden und dann durch Kaltschmieden bis zur Erhaltung der endgültigen Abmessungen des gewünschten Werkstückes behandelt und dass man an diesem Werkstück die längsverlaufenden Kanellierungen durch Elektrobearbeitung verwirklicht, die das gewünschte Querschnittsprofil (30) aufweisen und deren nichtzylindrische, längliche Innenfläche eine Form aufweist, die sich von dem unteren Ende bis zu dem oberen Ende verjüngt.

7. Verfahren nach Anspruch 6, dadurch gekennzeichnet, dass man bei der Elektrobearbeitung wenigstens eine Rinne mit Hilfe von zwei Elektroden (24, 25) verwirklicht, die in einer konvergierenden Bewegung (26) eintauchend arbeiten und ein Profil aufweisen, das demjenigen der Kanellierungen entspricht.

8. Verfahren nach einem der Ansprüche 6 oder 7, dadurch gekennzeichnet, dass man die Hälften (21a, 21b) des Prothesenschaftes (21) nacheinander bearbeitet, wobei die Prothese horizontal in einem Werkzeug gehalten wird.

9. Verfahren zur Herstellung einer Prothese nach einem der Ansprüche 1 bis 4 durch Warmschmieden eines zylindrischen Stangenstückes.

## Claims

1. Bone prosthesis intended to be attached without cement, and possessing, on its portion which is intended to be inserted into the bone, an external surface channelled in a direction generally parallel to its median axis, the said prosthesis being characterized in that:

the internal surface of the channels has a transverse sectional profile incorporating a narrowed portion (10 Figure 4) which broadens out towards the bottom (9) of the channel, this profile providing for the retention of the boney tissue which has developed inside the channel; and

the shape of the non-cylindrical longitudinal internal surface of the channels becomes thinner from the so-called bottom end (4) of the prosthesis, that is to say the end which is inserted more deeply into the bone, to the other, so-called top end (3), the prosthesis thereby being able to be extracted from the boney tissue by a pulling movement.

2. Prosthesis according to Claim 1, characterized in that the transverse sectional profile of the channels is curvilinear or circular or a combination of these two types of profile, the arcs being able to be connected by rectilinear portions.

3. Prosthesis according to one of Claims 1 and 2, characterized in that the transverse dimension of the interior of a channel at the top end (3) is less than that of the bottom end (4).

4. Prosthesis according to Claim 3, characterized in that the ratio between the transverse dimensions of the top end and the bottom end is between 0.99 and 0.099.

5. Process for producing an item of channelled prosthesis, according to any one of Claims 1 to 4, by the general technique of cire perdue casting in which a wax model is made corresponding to the item to be manufactured, refractory moulding is performed on the said model to form a mould and liquid metal is cast in this mould to produce the desired item, the said process being characterized in that, before the refractory moulding, rods (17) made, for example, of wax or polystyrene and having the transverse profile which corresponds to that of the channels in the final item, and which possess slight conicity from one end to the other, are placed in the channels (16) of the wax model so as to produce, after casting the metal in the refractory mould, an item with channels, the shape of whose non-cylindrical longitudinal internal surface becomes thinner from the bottom end to the top end.

6. Process for the production of an item of channelled prosthesis according to any one of Claims 1 to 4, characterized in that, by the known general technique of cire perdue casting, a smooth metal blank is made which possesses a slight overmeasure relative to the transverse dimensions of the item to be manufactured, in that the said blank is treated successively by hot forging and then by cold forging until the final dimensions of the desired item are obtained, and in that longitudinal channels (28, 29) having the desired transverse profile (30), and the shape of whose non-cylindrical longitudinal internal surface becomes thinner from the bottom end to the top end, are produced in the item by electroerosion.

7. Process according to Claim 6, characterized in that, during the electroerosion, at least one pass is made using two electrodes (24, 25) working in a dipping convergent movement (26) and having a profile corresponding to that of the channels.

8. Process according to one of Claims 6 and 7, characterized in that the procedure is carried out successively on the halves (21a, 21b) of the prosthesis shank (21), which is maintained horizontally in a tool (22).

9. Process for producing a prosthesis according to any one of Claims 1 to 4, by hot forging from a cylindrical bar.

0 098 224

FIG.1

FIG.2

FIG.3

FIG.4

FIG.5

FIG.6

FIG.7

FIG.8